# EUROPEAN PATENT APPLICATION

(11) **EP 4 821 553 A2**
(43) Date of publication of application: **14.10.2026**
(21) Application number: 26193333.7
(22) Date of filing: 04.08.2022
(51) Int. Cl.: A61P 1/16

(54) **CRYSTAL FORM OF LANIFIBRANOR, PREPARATION METHOD THEREFOR, AND USE THEREOF**

(30) Priority: 12.08.2021 CN 202110921824; 10.09.2021 CN 202111059866
(62) Document number(s) of the earlier application(s) in accordance with Art. 76 EPC: 22855302.0 / 4 385 990
(71) Applicant: Inventiva, 21121 Daix (FR)
(72) Inventor: CHEN, Minhua, Suzhou, Jiangsu, 215123 (CN); ZHU, Hongyan, Suzhou, Jiangsu, 215123 (CN); MENG, Liping, Suzhou, Jiangsu, 215123 (CN)
(74) Representative: Cabinet Beau de Loménie

(57) **Abstract**

The present disclosure provides a crystalline form of lanifibranor, a method for preparing said crystalline form as well as a pharmaceutical composition containing the crystalline form.

## Description

### TECHNICAL FIELD

The present disclosure pertains to the field of chemical crystallography, particularly relates to novel crystalline forms of Lanifibranor, preparation method and use thereof.

### BACKGROUND

Non-alcoholic steatohepatitis (NASH) is a severe liver disease with steatosis accompanied by inflammation and hepatocellular injury.

Peroxisome proliferator-activated receptor (PPAR) are ligand-activated transcription factors belonging to the nuclear hormone receptor family that regulate the expression of target genes. PPAR play essential roles in the regulation of cellular differentiation, development, and tumorigenesis.

Lanifibranor is an orally-available small molecule that acts to induce anti-fibrotic, anti-inflammatory by activating each of the three PPAR isoforms, known as PPARα, PPARδ and PPARγ. lanifibranor can addresses steatosis by enhancing fatty acid metabolism and ultimately decreasing lipogenesis.

The chemical name of Lanifibranor is 5-Chloro-1-[(6-benzothiazolyl)sulfonyl]-1H-indole-2-butanoic acid (Referred to as Compound I), and the structure is shown as follows:

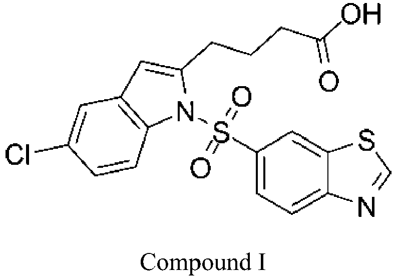

A crystalline form is a solid material whose constituents are arranged in a highly ordered microscopic structure, forming a crystal lattice that extends in all directions. Polymorphism refers to the phenomenon that a compound exists in more than one crystalline form. Compounds may exist in one or more crystalline forms, but their existence and characteristics cannot be predicted with any certainty. Different crystalline forms of drug substances have different physicochemical properties, which can affect drug's in vivo dissolution and absorption and will further affect drug's clinical efficacy and safety to some extent. In particular, for some poorly soluble oral solid or semisolid dosage forms, crystalline forms can be crucial to the performance of drug product. In addition, the physiochemical properties of a crystalline form are very important to the manufacturing process. Therefore, polymorphism is an important part of drug research and drug quality control.

Amorphous forms are non-crystalline materials which possess no long-range order. Typically, an amorphous form will exhibit a broad "halo" XRPD pattern.

Example 117 of prior art WO2007026097A1 disclosed the preparation of Compound I as: "the white precipitate is extracted with ethyl acetate and the organic phase is separated off, dried over magnesium sulfate and concentrated under reduced pressure to give Compound I in the form of a pale yellow powder (yield=83%)." Example 3 of prior art WO2019024776A1 disclosed the preparation of Compound I as: "the white precipitate is extracted with ethyl acetate and the organic phase is separated off, dried over magnesium sulfate and concentrated under reduced pressure to give 83 mg pale yellow solid of Compound I." The inventors of the present disclosure repeated the preparation method of Compound I disclosed in prior arts and obtained the amorphous of Compound I. The inventors of the present disclosure have systematically evaluated the properties of the amorphous obtained according to the prior arts. The results show that the amorphous has the problems of poor hygroscopicity, high organic solvent residue, poor powder condition, difficulty of quantification and large material loss during transfer and is not suitable for pharmaceutical use.

In order to overcome the disadvantages of prior arts, a new crystalline form meeting the medicinal standard is still needed for the development of drugs containing Compound I. The inventors of the present disclosure surprisingly obtained 1,4-dioxane solvate, chloroform solvate, tetrahydrofuran solvate, anhydrous and hydrate of Compound I. Among them, 1,4-dioxane solvate, trichloromethane solvate and tetrahydrofuran solvate contain 10.1 wt% of 1,4-dioxane, 3.3 wt% of trichloromethane and 6.0 wt% of tetrahydrofuran and are not suitable for pharmaceutical use. The anhydrate and hydrate of Compound I provided by the present disclosure have advantages in at least one aspect of solubility, hygroscopicity, purification ability, stability, adhesiveness, compressibility, flowability, in vitro and in vivo dissolution, and bioavailability, etc. In particular, the crystalline forms of the Compound I of the present disclosure have advantages such as good stability, good hygroscopicity, good flowability, low adhesiveness, good dispersion of crystalline powder, which solves the problems existing in the prior arts and are of great significance for the development of drugs containing Compound I.

### SUMMARY

The present disclosure is to provide novel crystalline forms of Compound I, preparation method and pharmaceutical compositions comprising the crystalline forms.

Furthermore, crystalline form CSV of Compound I is provided by the present disclosure (hereinafter referred to as Form CSV).

In one aspect provided herein, the X-ray powder diffraction pattern of Form CSV comprises characteristic peaks at 2theta values of 10.5°±0.2°, 11.4°±0.2° and 26.0°±0.2° using CuKα radiation.

Furthermore, the X-ray powder diffraction pattern of Form CSV comprises one or two or three characteristic peaks at 2theta values of 15.9°±0.2°, 18.0°±0.2° and 23.0°±0.2° using CuKα radiation. Preferably, the X-ray powder diffraction pattern of Form CSV comprises characteristic peaks at 2theta values of 15.9°±0.2°, 18.0°±0.2° and 23.0°±0.2° using CuKα radiation.

Furthermore, the X-ray powder diffraction pattern of Form CSV comprises one or two or three characteristic peaks at 2theta values of 25.0°±0.2°, 27.1°±0.2° and 28.9°±0.2° using CuKα radiation. Preferably, the X-ray powder diffraction pattern of Form CSV comprises characteristic peaks at 2theta values of 25.0°±0.2°, 27.1°±0.2° and 28.9°±0.2° using CuKα radiation.

In another aspect provided herein, the X-ray powder diffraction pattern of Form CSV comprises three or four or five or six or seven or eight or nine or ten or eleven or twelve or thirteen or fourteen or fifteen or sixteen characteristic peaks at 2theta values of 10.5°±0.2°, 11.4°±0.2°, 26.0°±0.2°, 15.9°±0.2°, 18.0°±0.2°, 23.0°±0.2°, 25.0°±0.2°, 27.1°±0.2°, 28.9°±0.2°, 5.7°±0.2°, 12.8°±0.2°, 21.0°±0.2°, 22.0°±0.2°, 23.6°±0.2°, 24.1°±0.2° and 26.6°±0.2° using CuKα radiation.

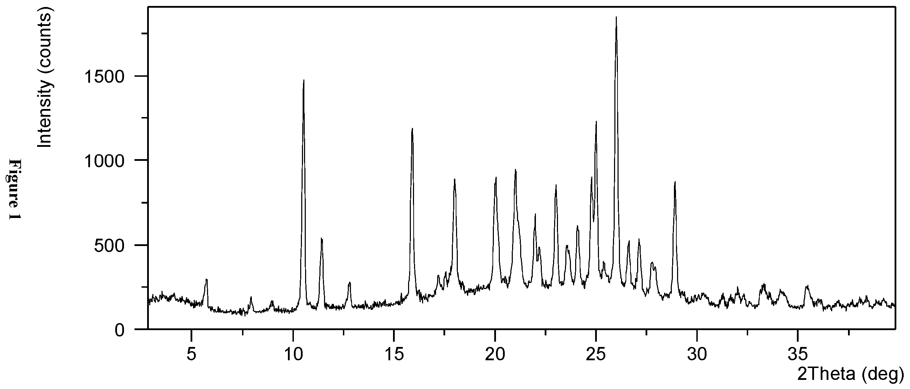

Without any limitation being implied, the X-ray powder diffraction pattern of Form CSV is substantially as depicted in Figure 1.

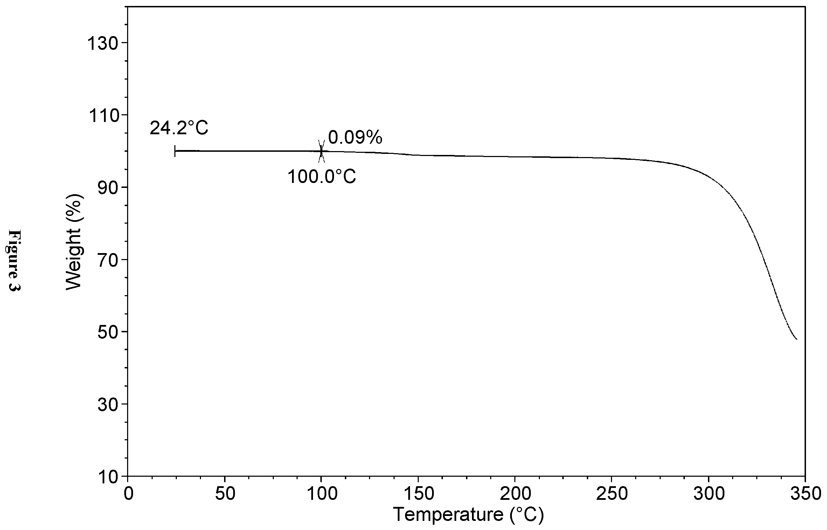

Without any limitation being implied, the Thermo Gravimetric Analysis (TGA) curve of Form CSV is substantially as depicted in Figure 3, which shows 0.1% weight loss when heated to 100 °C.

Without any limitation being implied, Form CSV is an anhydrate.

According to the objective of the present disclosure, a process for preparing Form CSV is also provided. The process comprises:
method 1: dissolving Compound I into an ether, filtering, putting the filtrate into a glass bottle, putting the system into a sealed glass bottle containing water to obtain Form CSV. or
method 2: dissolving Compound I into an ester, filtering, evaporating to obtain Form CSV.

Furthermore, in method 1, said ether is preferably a C4-C6 ether, further preferably 2-methyltetrahydrofuran.

Furthermore, in method 2, said ester is preferably a C2-C7 ester, further preferably isopropyl acetate. said evaporation temperature is preferably room temperature to 80 °C.

According to the objective of the present disclosure, crystalline form CSIII of Compound I is provided by the present disclosure (hereinafter referred to as Form CSIII).

In one aspect provided herein, the X-ray powder diffraction pattern of Form CSIII comprises characteristic peaks at 2theta values of 9.9°±0.2°, 15.7°±0.2° and 17.3°±0.2° using CuKα radiation.

Furthermore, the X-ray powder diffraction pattern of Form CSIII comprises one or two or three characteristic peaks at 2theta values of 11.7°±0.2°, 24.0°±0.2° and 26.7°±0.2° using CuKα radiation. Preferably, the X-ray powder diffraction pattern of Form CSIII comprises characteristic peaks at 2theta values of 11.7°±0.2°, 24.0°±0.2° and 26.7°±0.2° using CuKα radiation.

Furthermore, the X-ray powder diffraction pattern of Form CSIII comprises one or two or three characteristic peaks at 2theta values of 26.0°±0.2°, 28.3°±0.2° and 30.4°±0.2° using CuKα radiation. Preferably, the X-ray powder diffraction pattern of Form CSIII comprises characteristic peaks at 2theta values of 26.0°±0.2°, 28.3°±0.2° and 30.4°±0.2° using CuKα radiation.

In another aspect provided herein, the X-ray powder diffraction pattern of Form CSIII comprises three or four or five or six or seven or eight or nine or ten or eleven or twelve or thirteen or fourteen or fifteen characteristic peaks at 2theta values of 9.9°±0.2°, 15.7°±0.2°,17.3°±0.2°, 11.7°±0.2°, 24.0°±0.2°, 26.7°±0.2°, 26.0°±0.2°, 28.3°±0.2°, 30.4°±0.2°, 7.8°±0.2°, 18.0°±0.2°, 21.5°±0.2°, 23.4°±0.2°, 25.1°±0.2° and 27.0°±0.2° using CuKα radiation.

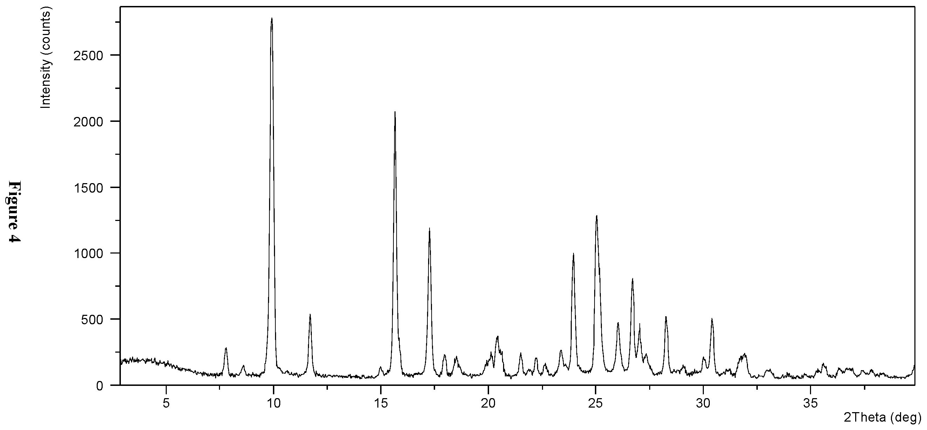

Without any limitation being implied, the X-ray powder diffraction pattern of Form CSIII is substantially as depicted in Figure 4.

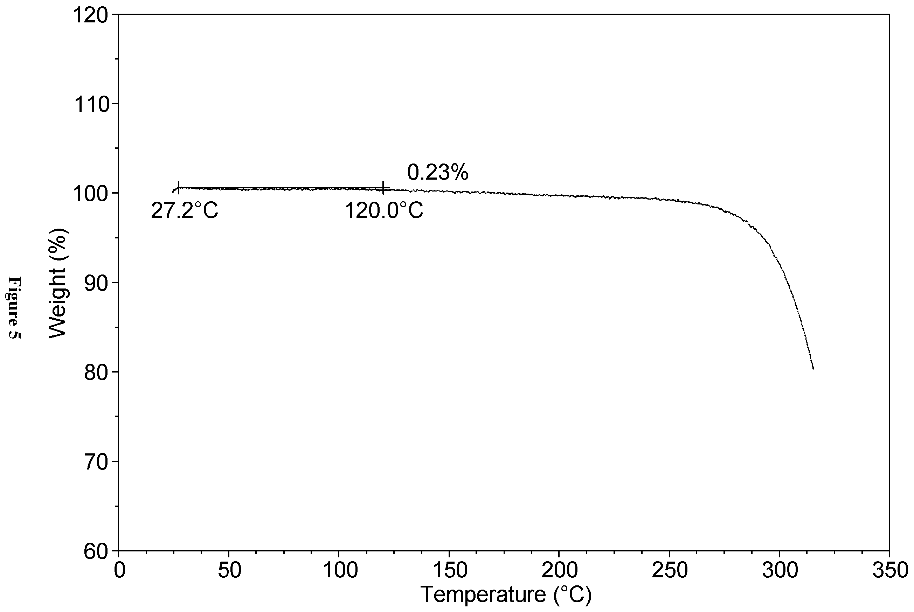

Without any limitation being implied, the Thermo Gravimetric Analysis (TGA) curve of Form CSIII is substantially as depicted in Figure 5, which shows 0.2% weight loss when heated to 120 °C.

Without any limitation being implied, Form CSIII is an anhydrate.

According to the objective of the present disclosure, a process for preparing Form CSIII is also provided. The process comprises:
Dissolving Compound I into a nitrile, filtering, evaporating to obtain Form CSIII.

Furthermore, said nitrile is preferably a C2-C4 nitrile, more preferably acetonitrile.

According to the objective of the present disclosure, the present disclosure also provides the use of Form CSV or Form CSIII or combinations thereof for preparing other crystalline forms or salts of Compound I.

According to the objective of the present disclosure, a pharmaceutical composition is provided, said pharmaceutical composition comprises a therapeutically effective amount of the crystalline form of Compound I and pharmaceutically acceptable excipients.

Furthermore, a pharmaceutical composition is provided, said pharmaceutical composition comprises a therapeutically effective amount of Form CSV, Form CSIII or combinations thereof and pharmaceutically acceptable excipients.

According to the objective of the present disclosure, the crystalline forms of Compound I can be used for preparing pan-PPAR agonist drugs.

Furthermore, Form CSV, Form CSIII or combinations thereof can be used for preparing pan-PPAR agonist drugs.

According to the objective of the present disclosure, the crystalline formsof Compound I can be used for preparing drugs treating NASH.

Furthermore, Form CSV, Form CSIII or combinations thereof can be used for preparing drugs treating NASH.

### TECHNICAL PROBLEMS SOLVED BY THE PRESENT DISCLOSURE

The inventors of the present disclosure have systematically evaluated the properties of the amorphous obtained according to the prior arts and the results show that the amorphous has poor hygroscopicity and poor chemical stability, which is not conducive to production. Meanwhile, the amount of organic solvent residues in the amorphous is far beyond the solvent residue limit of the Pharmacopoeia, and the toxicity is high. In order to solve the problems existing in the prior arts, the inventors of the present disclosure have conducted more than 600 experiments with different methods, such as slurry, volatilization, liquid vapor diffusion, solid vapor diffusion, humidity induction, anti-solvent addition and heating in different solvent systems, and finally unexpectedly obtained crystalline forms of Compound I provided by the present disclosure.

### TECHNICAL EFFECTS

Form CSV of the present disclosure has the following advantages:
(1) Compared with prior arts, Form CSV of the present disclosure has better hygroscopicity. The test results show that the weight gain of prior art amorphous from 0% to 70% relative humidity (RH) is about 3.4 times that of Form CSV. The weight gain of Form CSV from 0% to 70%RH is 0.35%, the weight gain of prior art amorphous from 0% to 70%RH is 1.18%, respectively.

In one aspect, poor hygroscopicity tends to cause chemical degradation and polymorph transformation, which directly affects the physicochemical stability of the drug substance. In addition, poor hygroscopicity will reduce the flowability of the drug substance, thereby affecting the processing of the drug substance.

In another aspect, drug substance with poor hygroscopicity requires low humidity environment during production and storage, which puts strict requirements on production and imposes higher costs. More importantly, poor hygroscopicity is likely to cause variation in the content of active pharmaceutical ingredients in the drug product, thus affecting drug product quality.

Form CSV provided by the present disclosure with good hygroscopicity is not demanding on the production and storage conditions, which reduces the cost of production, storage and quality control, and has strong economic value.

(2) Form CSV provided by the present disclosure has good appearance. Form CSV has good powder dispersibility, which is beneficial to transfer and reduce material loss in industrial production.

(3) From CSV drug substance of the present disclosure has good stability. Crystalline state of Form CSV drug substance doesn't change for at least 9 months when stored under the condition of 40 °C/75%RH with open and sealed package. The crystalline form of Form CSV drug substance doesn't change for at least 1 months when stored under the condition of 60 °C/75%RH with open and sealed package. These results show that Form CSV drug substance has good stability under accelerated and stress conditions. Drug substance would go through high temperature and high humidity conditions caused by different season, regional climate and environment during storage, transportation, and manufacturing processes. Therefore, good stability under accelerated and stress conditions is of great importance to the drug development. Form CSV drug substance has good stability under stress condition, which is beneficial to avoid the impact on drug quality due to crystal transformation or decrease in purity during drug storage.

(4) Form CSV of the present disclosure has good humidity stability. The crystalline form of Form CSV doesn't change after under a humidity cycle from 0%-95%-0%RH.

(5) Form CSV of the present disclosure has good flowability. Better flowability can prevent clogging of production equipment and increase manufacturing efficiency. Better flowability of Form CSV ensures the content uniformity of the drug product, reduces the weight variation of the drug product and improves product quality.

(6) Form CSV of the present disclosure shows low adhesiveness. Low adhesiveness can reduce the agglomeration of drug substance and effectively improve the adhesion to roller and tooling during dry-granulation and compression process. It is conducive to the dispersion of drug substance with excipients and improving the blend uniformity of the mixing of materials, which ultimately improves product quality.

### BRIEF DESCRIPTION OF THE DRAWINGS

Figure 1 shows an XRPD pattern of Form CSV according to example 2.
Figure 2 shows an XRPD pattern of Form CSV according to example 3.
Figure 3 shows a TGA curve of Form CSV.
Figure 4 shows an XRPD pattern of Form CSIII according to example 5.
Figure 5 shows a TGA curve of Form CSIII according to example 5.
Figure 6 shows an XRPD pattern of amorphous according to example 6.
Figure 7 shows a TGA curve of amorphous according to example 6.
Figure 8 shows an XRPD pattern overlay of Form CSV before and after storage under different conditions (from top to bottom: initial, 40 °C/75%RH for 9 months with open package, 40 °C/75%RH for 9 months with sealed package, 60 °C/75%RH for 1 month with open package, 60 °C/75%RH for 1 month with sealed package.)
Figure 9 shows an XRPD pattern overlay of Form CSV before and after DVS test (top: before test, bottom: after test)
Figure 10 shows a DVS curve of Form CSV.
Figure 11 shows the appearance of Form CSV powder.
Figure 12 shows an XRPD pattern overlay of Form CSV before and after tableting (from top to bottom: initial, 5 kN, 10 kN, 20 kN).
Figure 13 shows an XRPD pattern overlay of Form CSIII before and after DVS test (top: before test, bottom: after test)
Figure 14 shows the appearance of Form CSIII powder.

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.

The abbreviations used in the present disclosure are explained as follows:
XRPD: X-ray Powder Diffraction
TGA: Thermo Gravimetric Analysis
DSC: Differential Scanning Calorimetry
¹H NMR: Proton Nuclear Magnetic Resonance
DVS: Dynamic Vapor Sorption
HPLC: High Performance Liquid Chromatography
RH:Relative humidity

### Instruments and methods used for data collection:

X-ray powder diffraction patterns in the present disclosure were acquired by a Bruker X-ray powder diffractometer. The parameters of the X-ray powder diffraction method of the present disclosure are as follows:

| | | | |
|---|---|---|---|
| X-Ray source: | Cu, Kα | | |
| Kα1 (Å): | 1.54060; | Kα2 (Å): | 1.54439 |
| Kα2/Kα1 intensity ratio: | 0.50 | | |

Thermo gravimetric analysis (TGA) data in the present disclosure were acquired by a TA Q500. The parameters of the TGA method of the present disclosure are as follows:
Heating rate: 10 °C/ min
Purge gas: N₂

Differential scanning calorimetry (DSC) data in the present disclosure were acquired by a TA Q2000. The parameters of the DSC method of the present disclosure are as follows:
Heating rate: 10 °C/ min
Purge gas: N₂

Proton nuclear magnetic resonance spectrum data (¹H NMR) were collected from a Bruker Avance II DMX 400M Hz NMR spectrometer. 1-5 mg of sample was weighed and dissolved with 0.5 mL of deuterated dimethyl sulfoxide to obtain a solution with a concentration of 2-10 mg/mL.

Dynamic Vapor Sorption (DVS) was measured via an SMS (Surface Measurement Systems Ltd.) intrinsic DVS instrument. The instrument control software is DVS-Intrinsic control software. Typical Parameters for DVS test are as follows:
Temperature: 25 °C
Gas and flow rate: N₂, 200 mL/min
RH range: 0% RH to 95% RH

The parameters of related substance detection in the present disclosure are shown in Table 1.

**Table 1**

| | | |
|---|---|---|
| Instrument | Waters ACQUITY H-Class with PDA detector | |
| Column | ACE Excel 3 C18, 4.6*100 mm, 3.0 µm | |
| Mobile phase | A: Acetonitrile: Water (pH3.0, phosphoric acid) = 50:950 (v/v) | |
| | B: Acetonitrile | |
| Gradient | Time (min) | %A |
| | 0.0 | 60 |
| | 0.5 | 60 |
| | 5.0 | 20 |
| | 7.0 | 10 |
| | 12.0 | 10 |
| | 12.1 | 60 |
| | 18.0 | 60 |
| Run time | 18.0 min | |
| Post time | 0 min | |
| Flow rate | 1.0 mL/min | |
| Injection volume | 1 µL | |
| Detector wavelength | 220 nm | |
| Column temperature | 40 °C | |
| Sample temperature | Room Temperature | |
| Diluent | Acetonitrile | |

In the present disclosure, said "stirring" is accomplished by using a conventional method in the field such as magnetic stirring or mechanical stirring and the stirring speed is 50 to 1800 r/min. Preferably the magnetic stirring speed is 300 to 900 r/min and mechanical stirring speed is 100 to 300 r/min.

Said "separation" is accomplished by using a conventional method in the field such as centrifugation or filtration. The operation of "centrifugation" is as follows: the sample to be separated is placed into the centrifuge tube and then centrifuged at a rate of 10000 r/min until the solid all sink to the bottom of the tube.

Said "evaporating" is accomplished by using a conventional method in the field such as slow evaporation or rapid evaporation. Slow evaporation is accomplished in a container covered by a sealing film with pinholes. Rapid evaporation is accomplished in an open container.

The "concentrated under reduced pressure" accomplished by using a conventional method in the field. For example, the operation of concentrated under reduced pressure is to rotate the flask containing solution at a constant speed at a certain temperature and a certain reduced pressure to evaporate the solvent.

Said "room temperature" is not a specific temperature, but a temperature range of 10-30 °C.

Said "characteristic peak" refers to a representative diffraction peak used to distinguish crystals. The 2theta value of diffraction peak usually can have a deviation of ±0.2° using CuKα radiation.

In the present disclosure, "crystal" or "crystalline form" refers to the crystal or the crystalline form being identified by the X-ray diffraction pattern shown herein. Those skilled in the art are able to understand that the X-ray powder diffraction pattern depend on the instrument conditions, the sample preparation and the purity of samples. The relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CSV and Form CSIII of the present disclosure are pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

Unless otherwise specified, the following examples were conducted at room temperature.

According to the present disclosure, Compound I used as raw materials include, but are not limited to solid (crystalline and amorphous), semisolid, wax, oil, liquid form or solution. Preferably, Compound I used as the raw material is a solid.

Raw materials of Compound I used in the following examples were prepared by prior arts, for example, the method disclosed in WO2007026097A1.

### Example 1 Characterization of Compound I Solvates

1,4-dioxane solvate, chloroform solvate and tetrahydrofuran solvate of Compound I were characterized by TGA, DSC, and ¹H NMR. The results are listed in Table 2.

**Table 2**

| Form | Preparation method | TGA | DSC | ¹H NMR |
|---|---|---|---|---|
| 1,4-dioxane solvate | 23.8 mg of Compound I was weighed into a glass vial followed by adding 1 mL of 1,4-dioxane to dissolve the solid. The solution was filtered. The filtrate was divided into two parts, and one of the filtrate was put at 50 °C for rapid evaporation. | About 10.1% weight loss when heated to 200 °C. | Two endothermic peaks at around 130.1 °C and 178.2 °C, and an exothermic peak at around 135.8 °C. | 0.56 molar equivalents of 1,4-dioxane per mole of 1,4-dioxane solvate. |
| Chloroform solvate | 10.8 mg of 1,4-dioxane solvate was weighed into a glass vial followed by adding 0.3 mL of chloroform, and the system was stirred at -20 °C for one day. A solid was isolated and dried under vacuum at room temperature for 2 hours. | About 3.3% weight loss when heated to 230 °C. | Two endothermic peaks at around 96.8 °C and 179.2 °C, and an exothermic peak at around 122.8 °C. | 0.14 molar equivalents of chloroform per mole of chloroform solvate. |
| Tetrahydrofuran solvate | 9.5 mg of Compound I was weighed into a glass vial followed by adding 1 mL of tetrahydrofuran/chloroform (1:1, v/v) to dissolve the solid. The solution was filtered. The filtrate was put at 50 °C for slow evaporation. | About 6.0% weight loss when to 120 °C. heated | Three endothermic peaks around 103.7 °C, 168.4 °C and 179.2 °C, and two exothermic peaks at around 109.7 °C and 156.2 °C. | 0.37 molar equivalents of tetrahydrofuran and 0.05 molar equivalents of chloroform per mole of tetrahydrofuran solvate. |

Rapid evaporation: evaporate with open lid.

Slow evaporation: seal the container with aluminum foil, poke holes, and stand to evaporate.

### Example 2 Preparation of Form CSV

162.6 mg of Compound I was dissolved into 4.4 mL of 2-methyltetrahydrofuran. The solution was filtered. 0.3 mL of the filtrate was put into a glass bottle, then the glass bottle was put into a sealed glass bottle containing 4 mL of water. The solid form was obtained after the system stood for 12 days. The obtained solid was dried under vacuum at 30 °C overnight and at 60 °C for 4 days to obtain a dry solid.

The dry solid was confirmed to be Form CSV. The XRPD pattern is substantially as depicted in Figure 1, and the XRPD data are listed in Table 3.

**Table 3**

| Diffraction angle 2θ (°) | d spacing (Å) | Relative intensity (%) |
|---|---|---|
| 5.72 | 15.46 | 9.82 |
| 7.91 | 11.18 | 5.55 |
| 8.95 | 9.88 | 3.21 |
| 10.51 | 8.41 | 81.07 |
| 11.42 | 7.75 | 23.36 |
| 12.79 | 6.92 | 8.74 |
| 15.90 | 5.58 | 61.21 |
| 17.20 | 5.15 | 7.45 |
| 17.55 | 5.05 | 7.90 |
| 18.00 | 4.93 | 40.52 |
| 20.00 | 4.44 | 37.55 |
| 20.99 | 4.23 | 41.34 |
| 21.97 | 4.05 | 26.72 |
| 22.19 | 4.01 | 16.17 |
| 23.00 | 3.87 | 38.64 |
| 23.55 | 3.78 | 16.66 |
| 24.08 | 3.70 | 24.89 |
| 24.77 | 3.59 | 41.77 |
| 24.98 | 3.56 | 62.22 |
| 25.38 | 3.51 | 11.96 |
| 25.99 | 3.43 | 100.00 |
| 26.61 | 3.35 | 19.65 |
| 27.12 | 3.29 | 21.39 |
| 27.76 | 3.21 | 13.40 |
| 28.90 | 3.09 | 42.70 |
| 30.32 | 2.95 | 3.26 |
| 31.24 | 2.86 | 3.04 |
| 31.98 | 2.80 | 5.07 |
| 32.28 | 2.77 | 3.79 |
| 33.26 | 2.69 | 6.69 |
| 34.19 | 2.62 | 5.12 |
| 35.42 | 2.53 | 7.07 |
| 36.94 | 2.43 | 1.67 |

### Example 3 Preparation of Form CSV

143.7 mg of Compound I was dissolved into 120 mL of isopropyl acetate. The solution was filtered. 20 mL of filtrate was evaporated at 50 °C overnight to obtain the crystalline solid.

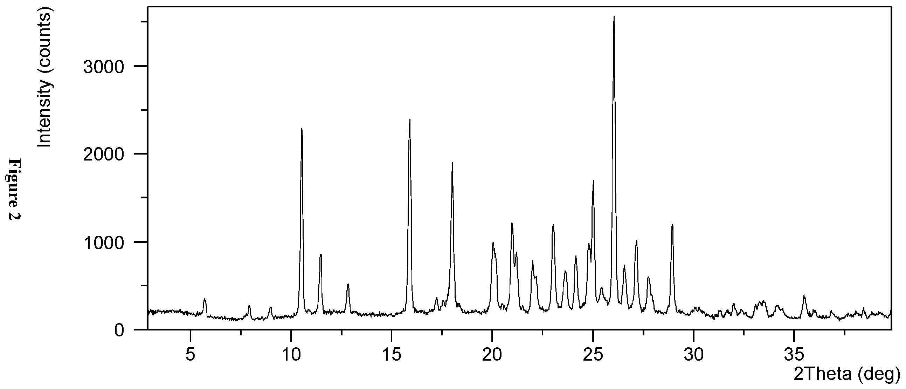

The crystalline solid was confirmed to be Form CSV. The XRPD pattern of Form CSV is substantially as depicted in Figure 2, and the XRPD data are listed in Table 4.

**Table 4**

| Diffraction angle 2θ (°) | d spacing (Å) | Relative intensity (%) |
|---|---|---|
| 5.70 | 15.51 | 5.34 |
| 7.91 | 11.18 | 4.59 |
| 8.96 | 9.87 | 3.66 |
| 10.51 | 8.41 | 62.83 |
| 11.44 | 7.73 | 20.93 |
| 12.81 | 6.91 | 10.67 |
| 15.88 | 5.58 | 65.20 |
| 17.22 | 5.15 | 5.87 |
| 18.00 | 4.93 | 50.72 |
| 20.02 | 4.44 | 24.53 |
| 20.16 | 4.40 | 19.77 |
| 20.96 | 4.24 | 31.24 |
| 21.17 | 4.20 | 21.17 |
| 21.98 | 4.04 | 18.36 |
| 22.13 | 4.02 | 12.78 |
| 23.02 | 3.86 | 30.52 |
| 23.62 | 3.77 | 15.01 |
| 24.14 | 3.69 | 20.20 |
| 24.79 | 3.59 | 24.22 |
| 25.00 | 3.56 | 45.32 |
| 25.42 | 3.50 | 9.51 |
| 26.03 | 3.42 | 100.00 |
| 26.55 | 3.36 | 17.25 |
| 27.14 | 3.29 | 24.39 |
| 27.75 | 3.22 | 13.25 |
| 28.92 | 3.09 | 31.19 |
| 30.13 | 2.97 | 2.38 |
| 31.29 | 2.86 | 1.91 |
| 31.99 | 2.80 | 4.39 |
| 33.49 | 2.68 | 5.21 |
| 34.15 | 2.63 | 4.08 |
| 35.49 | 2.53 | 7.43 |
| 35.99 | 2.50 | 2.28 |
| 36.86 | 2.44 | 1.87 |
| 38.45 | 2.34 | 2.10 |

### Example 4 The TGA of Form CSV

The TGA curve of Form CSV is substantially as depicted in Figure 3, which shows about 0.1% weight loss when heated to 100 °C.

### Example 5 Preparation of Form CSIII

5.2 mg of Compound I was weighed into 3.5 mL of acetonitrile. The system was dissolved at 50 °C, then the solution was filtered and evaporated at 50 °C to obtain a crystalline solid.

The crystalline solid was confirmed to be Form CSIII. The XRPD pattern of Form CSIII is substantially as depicted in Figure 4, and the XRPD data are listed in Table 5.

Form CSIII was dried under vacuum at 50 °C overnight and the crystalline form of Form CSIII was confirmed by XRPD after drying. No crystal transformation of Form CSIII was observed before and after drying. The TGA curve of Form CSIII after drying is substantially as depicted in Figure 5, which shows about 0.2% weight loss when heated to 120 °C.

The ¹H NMR data of Form CSIII are: ¹H NMR (400 MHz, DMSO-d6) δ 12.14 (s, 1H), 9.66 (s, 1H), 8.98 (d, J=1.8 Hz, 1H), 8.20 (d, J = 8.7 Hz, 1H), 8.09 (d, J = 8.9 Hz, 1H), 7.85 (dd, J=8.7, 2.0 Hz, 1H), 7.57 (d, J=2.1 Hz, 1H), 7.32 (dd, J=8.9, 2.1 Hz, 1H), 6.62 (s, 1H), 3.09 (t, J=7.4 Hz, 2H), 2.36 (t, J=7.3 Hz, 2H), 1.99-1.90 (m, 2H)_{∘} ¹H NMR results show that Form CSIII contains no residual solvent, while the prior art amorphous contains 2.70 wt% of ethyl acetate which far exceeds the residual solvent limit (0.5 wt %) specified in the Pharmacopoeia. Ethyl acetate has toxic side effects on the central nervous system. Long-term exposure can cause conjunctival irritation and corneal opacity and even cause liver and kidney congestion.

**Table 5**

| Diffraction angle 2θ (°) | d spacing (Å) | Relative intensity (%) |
|---|---|---|
| 7.78 | 11.36 | 7.20 |
| 8.58 | 10.31 | 2.49 |
| 9.91 | 8.92 | 100.00 |
| 11.70 | 7.57 | 16.18 |
| 15.00 | 5.91 | 2.69 |
| 15.65 | 5.66 | 74.14 |
| 17.26 | 5.14 | 42.11 |
| 17.96 | 4.94 | 6.15 |
| 18.49 | 4.80 | 5.39 |
| 19.98 | 4.44 | 4.16 |
| 20.40 | 4.35 | 11.43 |
| 21.49 | 4.14 | 6.41 |
| 22.20 | 4.00 | 5.17 |
| 22.62 | 3.93 | 3.46 |
| 23.38 | 3.80 | 7.60 |
| 23.95 | 3.72 | 34.43 |
| 25.03 | 3.56 | 45.51 |
| 26.04 | 3.42 | 15.39 |
| 26.70 | 3.34 | 27.82 |
| 27.02 | 3.30 | 13.22 |
| 27.36 | 3.26 | 6.30 |
| 28.26 | 3.16 | 17.16 |
| 29.06 | 3.07 | 2.94 |
| 30.02 | 2.98 | 5.30 |
| 30.40 | 2.94 | 15.94 |
| 31.13 | 2.87 | 2.04 |
| 31.92 | 2.80 | 6.30 |
| 33.04 | 2.71 | 1.97 |
| 35.61 | 2.52 | 3.22 |
| 36.32 | 2.47 | 2.08 |
| 36.78 | 2.44 | 1.85 |

### Example 6 Repeat the preparation method of Compound I disclosed in the prior art

According to Table 6, a certain amount of Compound I solid was dissolved by a certain volume of ethyl acetate to dissolve the solid. The solution was filtered. A certain volume of the filtrate was concentrated under reduced pressure at a certain temperature to obtain a solid. The obtained solid was labeled as Samples 1-2.

**Table 6**

| Sample | Weight of Compound I (mg) | Ethyl acetate volume (mL) | Filtrate volume(mL) | Temperature of concentration (°C) | Vacuum drying under 30 °C | Sample appearance | Solid form |
|---|---|---|---|---|---|---|---|
| 1 | 250.1 | 80 | 30 | 40 | Yes | Flocculent powder | Amorphous |
| 2 | 250.1 | 80 | 16 | 40 | No | Transparent gelatinous solid | Amorphous |

Samples 1-2 were confirmed by XRPD to be amorphous.

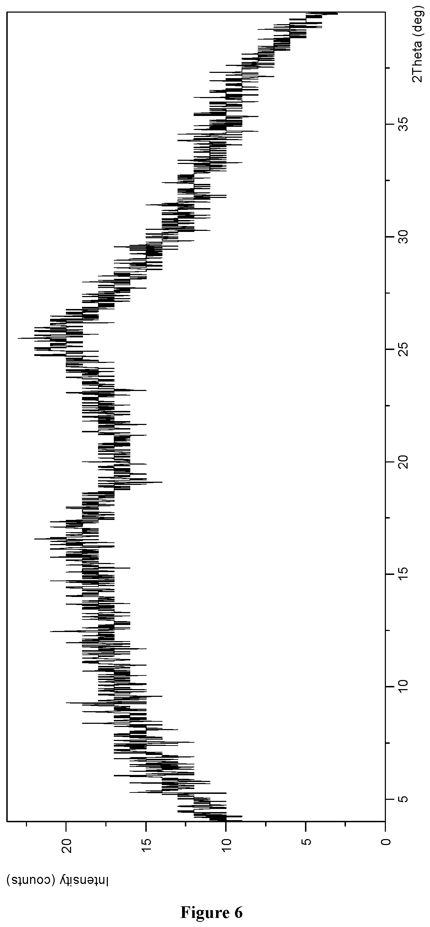

The XRPD pattern of Sample 1 is substantially as depicted in Figure 6.

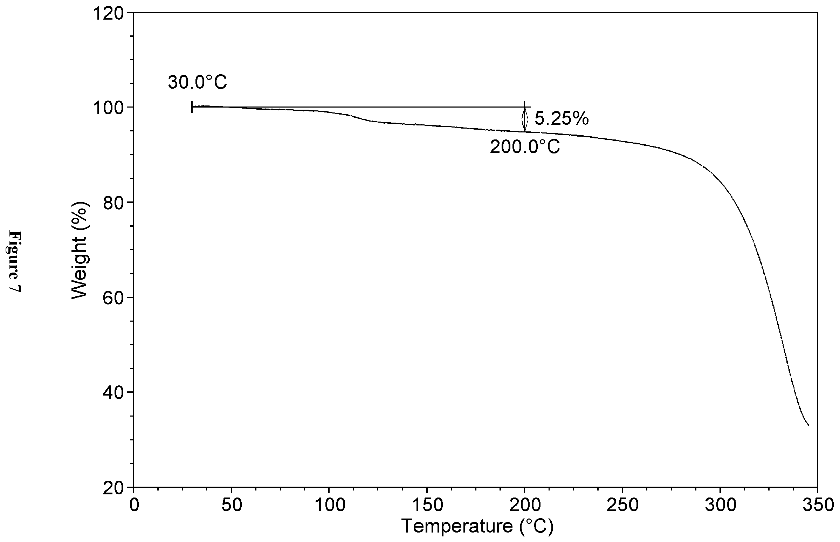

The TGA curve of Sample 1 is substantially as depicted in Figure 7, which shows about 5.3% weight loss when heated to 200 °C.

The ¹H NMR data of Sample 1 are: ¹H NMR (400 MHz, DMSO-d6) δ(ppm) 12.11 (s, 1H), 9.65 (s, 1H), 8.97 (d, *J* = 2.0 Hz, 1H), 8.20 (d, J = 8.8 Hz, 1H), 8.09 (d, *J* = 8.9 Hz, 1H), 7.84 (dd, *J* = 8.7, 2.1 Hz, 1H), 7.57 (d, *J* = 2.1 Hz, 1H), 7.31 (dd, *J* = 8.9, 2.3 Hz, 1H), 6.61 (s, 1H), 3.08 (t, *J* = 14.8 Hz, 1H), 2.36 (t, *J* = 7.3 Hz, 2H), 1.97-1.90 (m, 2H). The NMR signals at (δ(ppm) 4.03 (q, *J* = 7.1 Hz), 1.17 (t, *J* = 7.1 Hz, 0.58H)) belong to ethyl acetate. NMR results show that the prior art amorphous contains 2.70 wt% of ethyl acetate.

### Example 7 Physical and chemical stability of Form CSV

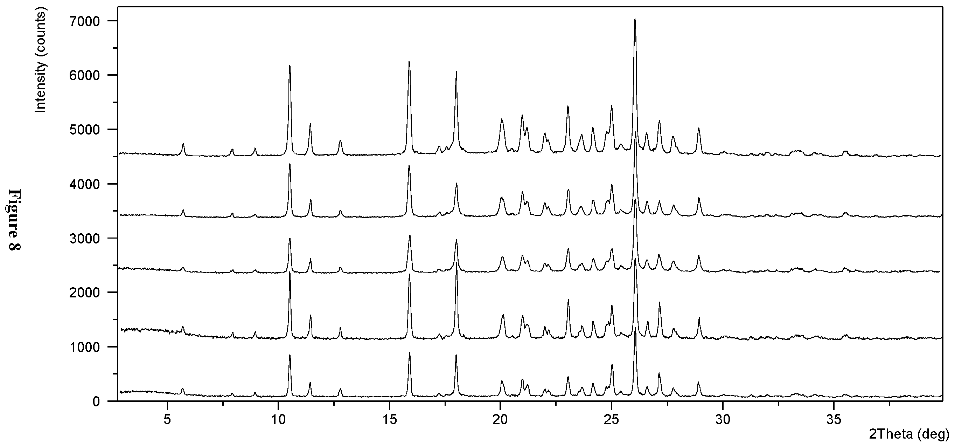

A certain amount of Form CSV of the present disclosure was stored under different conditions of 40 °C/75%RH and 60 °C/75%RH. Crystalline form and chemical purity were checked by XRPD and HPLC, respectively. The results are shown in Table 7 and the XRPD overlay is shown in Figure 8.

**Table 7**

| Initial | Conditions | Packing conditions | Time | Form |
|---|---|---|---|---|
| | Initial | N/A | - | Form CSV |
| CSV | 40 °C/75%RH | Sealed packaged | 9 months | Form CSV |
| | 40 °C/75%RH | Open packaged | 9 months | Form CSV |
| | 60 °C/75%RH | Sealed packaged | 1 month | Form CSV |
| | 60 °C/75%RH | Open packaged | 1 month | Form CSV |

Open packaged: Put the sample into a glass vial, cover the vial with aluminum foil, and punch 5-10 holes in the foil.

Sealed packaged: Put the sample into a glass vial, and seal the vial and desiccant in an aluminum foil bag.

The results show that Form CSV is stable for at least 9 months under 40 °C/75%RH. It shows that Form CSV has good stability under accelerated condition. Form CSV is stable for at least 1 month under 60 °C/75%RH. It shows that Form CSV has good stability under more stress conditions.

### Example 8 Hygroscopicity of amorphous

Dynamic vapor sorption (DVS) analyzer was applied to evaluate the hygroscopicity of amorphous with a certain amount of sample obtained by Example 6. The weight gains at each relative humidity were recorded in a cycle of 0%-95%-0%RH. The results show that the weight gain of amorphous from 0% to70%RH is 1.18%.

### Example 9 Hygroscopicity of Form CSV

Dynamic vapor sorption (DVS) analyzer was applied to evaluate hygroscopicity of Form CSV with a certain amount of sample. The weight gains at each relative humidity were recorded in a cycle of 0%-95% -0%RH.

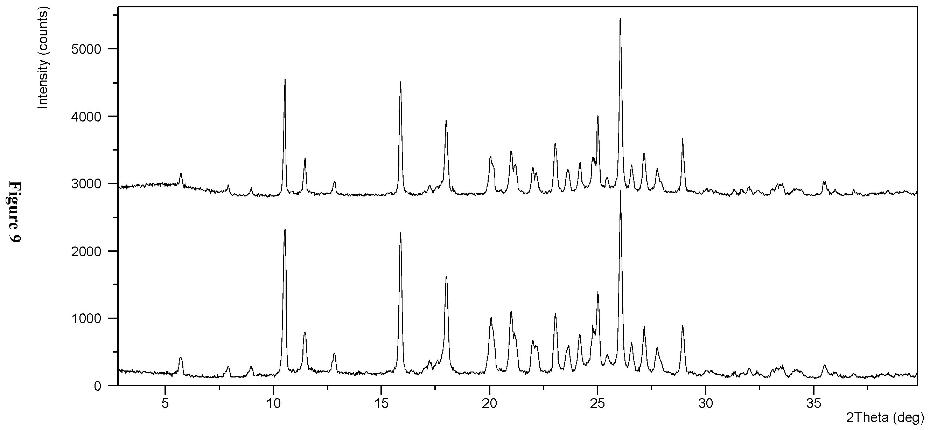

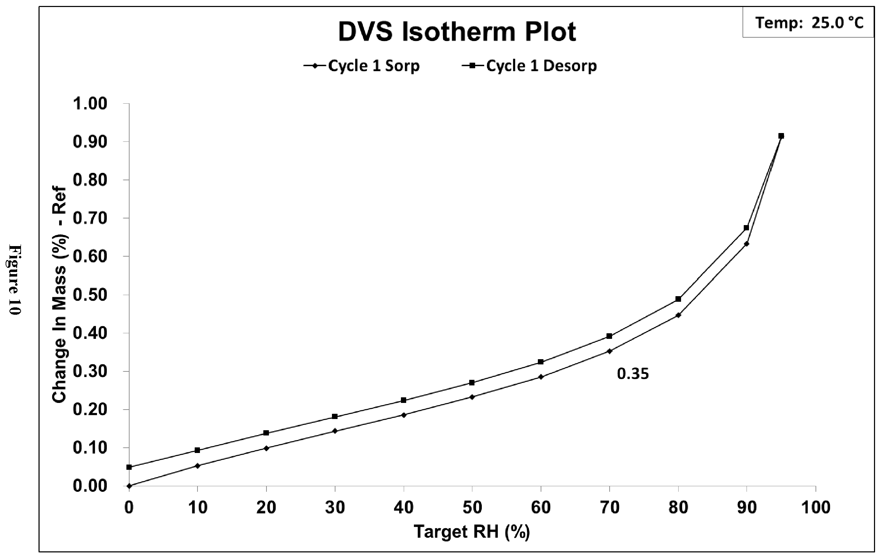

The results show that the weight gain of Form CSV from 0 to 70%RH is 0.35%. The weight gain of amorphous from 0 to 70%RH is about 3.4 times that of Form CSV. The XRPD patterns of Form CSV before and after DVS are shown in Figure 9 and the DVS curve of Form CSV is shown in Figure 10. The results show that the crystalline state of Form CSV remains unchanged after DVS.

### Example 10 Appearance of Form CSV

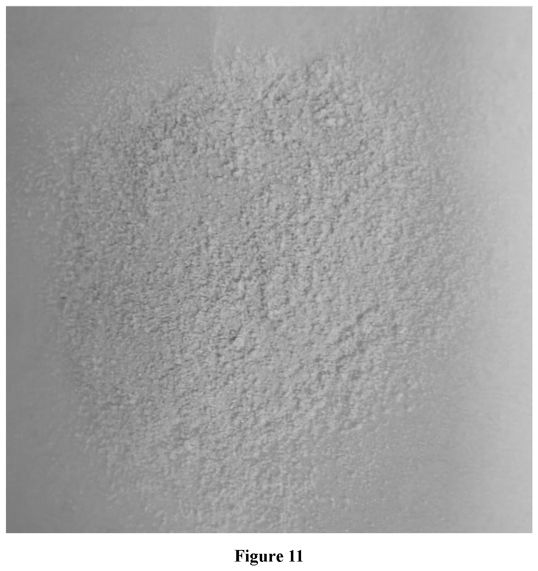

A certain amount of Form CSV was weighed and put on weighting paper to observe the appearance The appearance of Form CSV is shown in Figure 11. The result shows that Form CSV is powdery with uniformly dispersed and has no adhesion phenomenon.

### Example 11 Flowability of Form CSV

Compressibility index is usually utilized to evaluate the flowability of powder or granules during the drug product process. Compressibility index test method is as follows: a certain amount of powder was added into a measuring cylinder and bulk volume was recorded. Then the powder was tapped to make it in the tightest state and the tapped volume was recorded. The bulk density (ρ₀) and tapped density (ρf) were calculated and compressibility index was calculated according to c=(ρf-ρ₀)/ρf.

Criteria of flowability according to ICH Q4B Annex 13 is shown in Table 8.

**Table 8**

| Compressibility index (%) | Flowability |
|---|---|
| ≦10 | Excellent |
| 11-15 | Good |
| 16-20 | Fair |
| 21-25 | Passable |
| 26-31 | Poor |
| 32-37 | Very poor |
| >38 | Very, very poor |

Flowability test results of Form CSV is presented in Table 9, which indicate that Form CSV has good flowability.

**Table 9**

| Form | Bulk density (g/mL) | Tapped density (g/mL) | Flowability |
|---|---|---|---|
| Form CSV | 0.412 | 0.515 | Fair |

### Example 12 Adhesiveness of Form CSV

ENERPAC manual tablet press was used for testing. Approximately 30 mg of Form CSV was weighed and then added into the dies of ϕ8 mm round tooling, compressed at 10 kN and held for 30s. The punch was weighed and the amount of material sticking to the punch was calculated. The compression was repeated twice and the maximum amount and average amount of material sticking to the punch during the compression were recorded. The results show that the average adhesion amount of Form CSV was 0.45 mg. Form CSV shows low adhesiveness.

### Example 13 Physical stability of Form CSV upon mechanical force

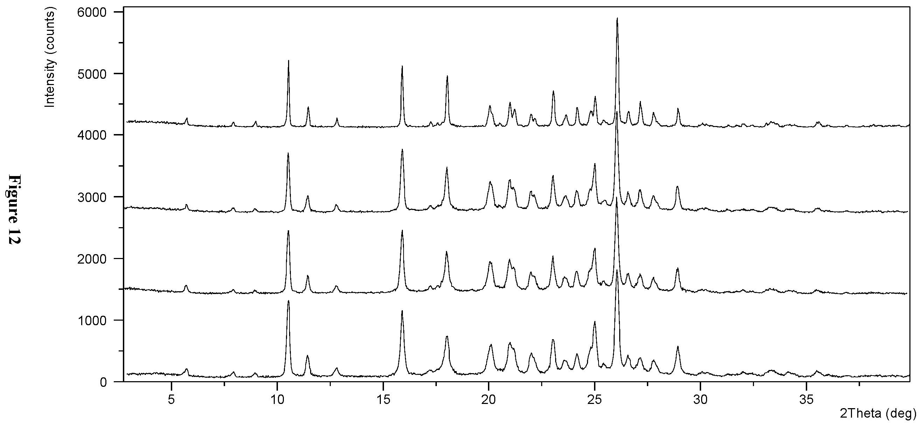

A certain amount of Form CSV was compressed into tablet under different pressures with suitable tableting die. Crystalline form before and after tableting were checked by XRPD. The test results are shown in Table 10, the XRPD pattern before and after tableting are shown in Figure 12. The results show that Form CSV has good stability under different pressures.

**Table 10**

| Before tableting | Pressure | Solid form after tableting |
|---|---|---|
| | 5 kN | Form CSV |
| Form CSV | 10 kN | Form CSV |
| | 20 kN | Form CSV |

### Example 14 Physical and chemical stability of Form CSIII

A certain amount of Form CSIII of the present disclosure was stored under different conditions of 25 °C/60%RH and 40 °C/75%RH with sealed and open packaged. Crystalline form and chemical purity were checked by XRPD and HPLC, respectively.

Open packaged: Put the sample into a glass vial, cover the vial with an aluminum foil, and punch 5-10 holes in the foil.

Sealed packaged: Put the sample into a glass vial, and seal the vial and desiccant in an aluminum foil bag.

The results show that Form CSIII is stable for at least 6 months under 25 °C/60%RH. The chemical purity is above 99.5% and remains substantially unchanged during storage. It shows that Form CSV has good stability under accelerated condition. Form CSIII is stable for at least 2 months under 40°C/75%RH. The chemical purity is above 99.5% and remains substantially unchanged during storage. It shows that Form CSIII has good stability under long and accelerated conditions.

### Example 15 Hygroscopicity of Form CSIII

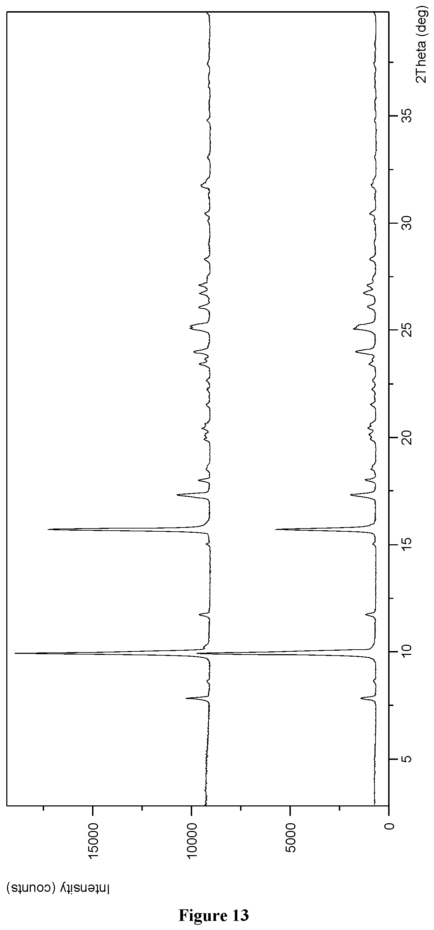

Dynamic vapor sorption (DVS) analyzer was applied to evaluate hygroscopicity of Form CSIII with a certain amount of sample. The weight gains at each relative humidity were recorded in a cycle of 0%-95% -0%RH. The XRPD patterns of Form CSIII before and after DVS test are shown in Figure 13 and the DVS curve of Form CSIII is shown in Figure 13. The results show that the crystalline state of Form CSIII remains unchanged after DVS.

### Example 16 Appearance of Form CSIII

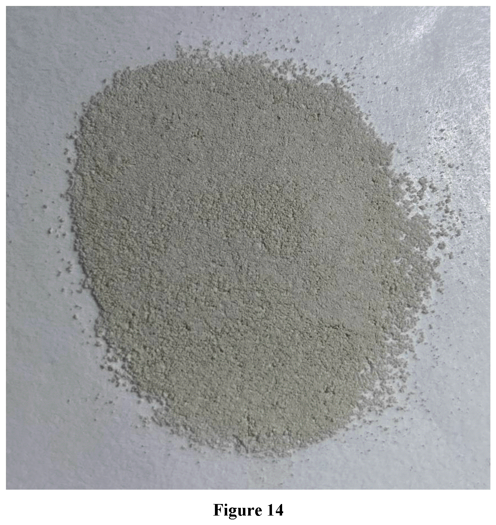

A certain amount of Form CSIII was weighed and put on a pan paper to observe the appearance The appearance of Form CSIII is shown in Figure 14. The result shows that Form CSIII is powdery and uniformly dispersed, and has no adhesion phenomenon, which is beneficial to transfer in industrial production and reducing material loss.

The examples described above are only for illustrating the technical concepts and features of the present disclosure and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A crystalline form of compound I, wherein the X-ray powder diffraction pattern comprises characteristic peaks at 2θ values of 10.5°±0.2°, 15.9°±0.2°, 18.0°±0.2°, 25.0°±0.2° and 26.0°±0.2°using Cu-Kα radiation

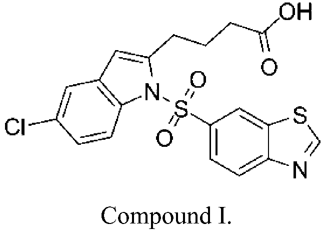

2. The crystalline form of Compound I according to claim 1, wherein the X-ray powder diffraction pattern further comprises at least one characteristic peak at 2θ values of 21.0°±0.2°, 23.0°±0.2° and 28.9°±0.2° using Cu-Kα radiation.

3. The crystalline form of Compound I according to claim 1 or claim 2, wherein the X-ray powder diffraction pattern further comprises at least one characteristic peak at 2θ values of 11.4°±0.2°, 20.0°±0.2°, 21.2°±0.2°, 24.1°±0.2°, 24.7°±0.2° and 27.1°±0.2 using Cu-Kα radiation.

4. The crystalline form of Compound I according to any one of claims 1 to 3, wherein the X-ray powder diffraction pattern is as depicted in Figure 1 using Cu-Kα radiation.

5. The crystalline form of Compound I according to any one of claims 1 to 3, wherein the X-ray powder diffraction pattern is as depicted in Figure 2 using Cu-Kα radiation.

6. A pharmaceutical composition comprising the crystalline form of Compound I as defined in any one of claims 1 to 5, and pharmaceutically acceptable excipients.

7. The crystalline form of Compound I as defined in any one of claims 1 to 5 for use as a medicament.

8. The crystalline form of Compound I as defined in any one of claims 1 to 5, or the pharmaceutical composition as defined in claim 6, for use in the treatment of non-alcoholic steatohepatitis.

9. Use of the crystalline form of Compound I as defined in any one of claims 1 to 5 for preparing other crystalline forms of Compound I.
